Europäisches Patentamt

European Patent Office

Office européen des brevets

(11) Numéro de publication: **0 258 079 B1**

# FASCICULE DE BREVET EUROPEEN

(45) Date de publication de fascicule du brevet: **19.02.92**  (51) Int. Cl.5: **C23G 5/028**, G03F 7/26

(21) Numéro de dépôt: **87401626.4**

(22) Date de dépôt: **09.07.87**

(54) **Composition à base de chlorure de méthylène- son utilisation pour l'enlèvement des films photoresist.**

(30) Priorité: **21.07.86 FR 8610529**

(43) Date de publication de la demande:
**02.03.88 Bulletin  88/09**

(45) Mention de la délivrance du brevet:
**19.02.92 Bulletin  92/08**

(84) Etats contractants désignés:
**AT BE CH DE FR GB GR IT LI LU NL SE**

(56) Documents cités:
**EP-A- 0 116 343**
**FR-A- 2 219 247**
**FR-A- 2 350 319**
**US-A- 3 625 763**
**US-A- 3 646 229**

(73) Titulaire: **ATOCHEM**
**4 & 8, Cours Michelet La Défense 10**
**F-92800 Puteaux(FR)**

(72) Inventeur: **Campos, Michel**
**36 rue de la Marne**
**F-78800 Houilles(FR)**
Inventeur: **Boussaguet, Jean-Charles**
**11 Rue Alphonse Daudet**
**F-78410 Aubergenville(FR)**
Inventeur: **Letullier, Jean-Philippe**
**15 Rue des Coutures**
**F-92190 Meudon(FR)**

**Description**

La présente invention concerne une composition à base de chlorure de méthylène et son utilisation pour enlever les films photoresist sur les circuits imprimés dans l'industrie électronique.

Une des étapes de la fabrication des circuits imprimés, consiste à enlever à l'aide d'un solvant, un film sec polymérisé sur l'épargne de gravure. Ce film sec ou "photoresist", qui a polymérisé sous un rayonnement ultra violet, résiste à l'eau, aux développeurs et aux agents de gravure, alors que les parties du film non isolées ont été dissoutes au cours de l'opération de développement.

On connait plusieurs compositions pour enlever ces films photoresist. Le brevet US 3,789,007 décrit un mélange de chlorure de méthylène contenant 3 à 15 % en poids de méthanol. Le brevet US 3,813,309 décrit un mélange de solvants chlorés contenant un acide organique fort, une amine et une cétone. Le brevet US 4,438,192 décrit du chlorure de méthylène contenant du méthanol, du formiate de méthyle et éventuellement de l'oxyde de propylène.

FR 2 219 247 divulgue, en page 9, du chlorure de méthylène ($CH_2Cl_2$) contenant de l'oxyde de propylène, de l'alcool méthylique, de l'acétone, de l'acétate d'amyle, de l'amylène, une amine et du N-méthylpyrrole. FR 2 350 319 divulgue du $CH_2Cl_2$ avec un époxyde et un éther. US 3 646 229 divulgue du $CH_2Cl_2$ avec du diméthoxyméthane. US 3 625 763 décrit du $CH_2Cl_2$ avec de l'alcool éthylique, de l'acétone, du chlorure d'éthyle et du trichloroéthane.

L'invention se propose de fournir une composition pour enlever facilement et rapidement les films photoresist sur les ébauches de circuits imprimés, ladite composition pouvant être recyclée et utilisée de nombreuses fois.

Cette composition est constituée par du chlorure de méthylène et :

a) au moins un époxyde

b) au moins un alcool

c) au moins un produit choisi parmi les éthers et les cétones peu solubles dans l'eau, les éthers ayant de 2 à 12 atomes de carbone et les cétones aliphatiques ayant de 6 à 10 atomes de carbone.

La quantité totale d'époxyde a) à utiliser peut être comprise entre 0,05 et 5 % en poids de la masse totale de la composition et de préférence entre 0,2 et 2 %. Les époxydes peuvent être l'oxyde de propylène et l'oxyde de butylène. On préfère l'oxyde de propylène.

Comme alcools, on utilise le plus souvent des alcools linéaires ou ramifiés ayant de 1 à 5 atomes de carbone et parmi eux, le méthanol, l'éthanol, et l'isopropanol. On préfère utiliser le méthanol.

La quantité totale d'alcools b) peut être comprise entre 0,05 et 5 % en poids de la masse totale de la composition, et de préférence entre 2 et 4 %.

La quantité totale de c) peut être comprise entre 0,05 et 5 % en poids de la masse totale de la composition, et de préférence entre 1 et 3 %.

Comme éthers, on utilise le plus souvent des mono, di ou triéthers ayant de 2 à 12 atomes de carbone. Le méthyl-tert-butyléther (MTBE) et le diméthoxyméthane conviennent particulièrement.

On peut utiliser un éther ou une cétone ou un mélange comprenant plusieurs produits appartenant à la même famille ou aux deux familles précitées. On utilise avantageusement le MTBE seul : le MTBE est peu soluble dans l'eau (1,3 % en poids à 20°C), ce qui est un avantage dans le cas où on régénère la composition par entraînement à la vapeur puisqu'on ne perd pas le produit.

La composition selon l'invention est préparée par simple mélange des produits avec le chlorure de méthylène. Ce mélange peut se faire à température ambiante et plus spécialement comprise entre 5 et 30°C.

La présente invention concerne aussi un procédé d'enlèvement des films photoresist à l'aide de la composition selon l'invention. L'enlèvement du film photoresist peut se faire par un simple trempage du circuit imprimé dans le liquide selon l'invention. On peut aussi utiliser un traitement continu par aspersion. Après l'enlèvement du film photoresist le circuit est égoutté et séché éventuellement à l'air.

Les exemples suivants illustrent l'invention sans la limiter. Dans les exemples suivants, le photoresist utilisé est une résine composée principalement d'un polyméthacrylate, et les concentrations sont en pourcentages pondéraux.

EXEMPLES 1 et 2

On prépare 2 compositions de formules suivantes :

|  | Composition 1 | Composition 2 |
|---|---|---|
| Chlorure de méthylène | 94 | 94 |
| Oxyde de propylène | 0,5 | 0,5 |
| Méthanol | 3 | 3 |
| Méthyl-tert-butyléther | 2,5 | 0 |
| Diméthoxyméthane | 0 | 2,5 |

On mesure d'abord la saturation des compositions en photoresist. A 100 g de composition, sont ajoutés quelques grammes de photoresist. Le tout est agité pendant 15 mn, puis on rajoute du photoresist et ainsi de suite jusqu'à saturation. Les essais sont effectués à 20°C. Après décantation, un extrait sec est réalisé sur une prise d'essai limpide. Les résultats sont regroupés dans le tableau I ci-après.

On mesure ensuite l'aptitude des compositions à être recyclées. Dans un ballon de laboratoire de 100 ml équipé pour la distillation des solvants ou charge la composition 1 contenant 3 % de photoresist, puis on distille pour recueillir la composition. Le distillat recueilli est alors "rechargé" avec 3 % de photoresist et distillé. Cette opération est effectuée 6 fois de suite. On effectue le même test pour la composition 2. Les résultats sont regroupés dans le tableau 1 ci-après.

Vitesse d'enlèvement du photoresist :

Des éprouvettes de cuivre de 10 x 1 cm revêtues de photoresist sont plongées dans les différentes formules et soumises à une agitation constante à une température de 25°C. Le temps de décapage complet d'une même surface d'éprouvette est mesuré.

Les essais ont été effectués avec des compositions neuves et des compositions ayant subi 6 recyclages dans le test précédent. Les résultats sont reportés sur le tableau I.

EXEMPLE 3

On mesure l'aptitude des compositions à être régénérées par entraînement à la vapeur. Dans cet exemple on compare une composition selon l'invention contenant du chlorure de méthylène du méthanol, de l'oxyde de propylène et du MTBE (composition 4) avec une composition non conforme à l'invention et contenant du chlorure de méthylène, du méthanol, de l'oxyde de propylène et du formiate de méthyle (composition 3).

Dans un appareillage d'entraînement à la vapeur 100 ml de composition contenant 3 % en poids de photoresist sont entraînés. Le condensat est alors rechargé de 3 % de photoresist puis à nouveau entraîné, cette opération est effectuée 4 fois de suite. Ces résultats sont regroupés dans le tableau II. La formule au MTBE est beaucoup moins altérée que l'autre. Il suffit de rajouter du méthanol pour obtenir une composition fraiche utilisable.

EP 0 258 079 B1

TABLEAU I

|  | Composition 1 | Composition 2 |
|---|---|---|
| Saturation en photoresist à 20°C en g/100 g | 7 | 6 |
| Formule de la composition après 6 distillations en présence de photoresist : | | |
| chlorure de méthylène | 96,08 | 95,3 |
| oxyde de propylène | 0,44 | 0,35 |
| méthanol | 3,34 | 4,21 |
| MTBE | 1,4 | – |
| diméthoxyméthane | – | 1,4 |
| Durée d'un décapage complet en secondes à 25°C | | |
| – avec une composition neuve | 17 | 16 |
| – avec une composition ayant subi 6 recyclages | 15 | 15 |

4

TABLEAU II

| | Composition 3 | | | Composition 4 | | |
|---|---|---|---|---|---|---|
| | oxyde de propylène | $CH_3OH$ | Formiate méthyle | oxyde de propylène | $CH_3OH$ | MTBE |
| Concentration initiale (dont chlorure de méthylène 94 %) | 0,5 | 3 | 2,5 | 0,5 | 3 | 2,5 |
| Concentration après le 1er entraînement | 0,53 | 0,16 | 2,21 | 0,53 | 0,48 | 2,95 |
| Concentration après le 2ème entraînement | 0,5 | 0,1 | 2 | 0,56 | 0,06 | 3,49 |
| Concentration après le 3ème entraînement | 0,52 | 0,016 | 1,79 | 0,54 | 0,016 | 3,7 |
| Concentration après le 4ème entraînement | 0,53 | 0 | 1,7 | 0,5 | 0,014 | 3,4 |

**Revendications**

1. Composition utile pour l'enlèvement des films photoresist, caractérisée en ce qu'elle est constituée de chlorure de méthylène, et de

5

a) au moins un époxyde

b) au moins un alcool

c) au moins un produit choisi parmi les éthers et les cétones peu solubles dans l'eau, les éthers ayant de 2 à 12 atomes de carbone et les cétones aliphatiques ayant de 6 à 10 atomes de carbone.

2. Composition selon la revendication 1, caractérisée en ce que l'époxyde est choisi parmi l'oxyde de propylène et l'oxyde de butylène.

3. Composition selon la revendication 1 ou 2, caractérisée en ce que l'alcool est choisi parmi les alcools linéaires ou ramifiés ayant de 1 à 5 atomes de carbone et est de préférence du méthanol.

4. Composition selon l'une des revendications 1 à 3, caractérisée en ce que l'éther est du méthyl-tert-butyléther ou du diméthoxyméthane.

5. Composition selon l'une des revendications 1 à 4, caractérisée en ce que la quantité totale de a) est comprise entre 0,05 et 5 % en poids de la masse totale de la composition et de préférence entre 0,2 et 2 %.

6. Composition selon l'une des revendications 1 à 5, caractérisée en ce que la quantité totale de b) est comprise entre 0,05 et 5 % en poids de la masse totale de la composition et de préférence entre 2 et 4 %.

7. Composition selon l'une des revendications 1 à 6, caractérisée en ce que la quantité totale de c) est comprise entre 0,05 et 5 % en poids de la masse totale de la composition et de préférence entre 1 et 3 %.

8. Composition selon l'une des revendications 1 à 7, caractérisée en ce que a) est de l'oxyde de propylène, b) du méthanol et c) du méthyl-tert-butyléther.

9. Procédé d'enlèvement de films photoresist sur des circuits imprimés, caractérisé en ce que lesdits circuits sont mis en contact par trempage ou aspersion avec une composition selon l'une des revendications 1 à 8.

**Claims**

1. Composition suitable for removing photoresist films, characterised in that it consists of methylene chloride and of

a) at least one epoxide

b) at least one alcohol

c) at least one product chosen from ethers and ketones which are poorly soluble in water, the ethers containing from 2 to 12 carbon atoms and the aliphatic ketones containing from 6 to 10 carbon atoms.

2. Composition according to Claim 1, characterised in that the epoxide is chosen from propylene oxide and butylene oxide.

3. Composition according to Claim 1 or 2, characterised in that the alcohol is chosen from linear or branched alcohols containing from 1 to 5 carbon atoms and is preferably methanol.

4. Composition according to one of Claims 1 to 3, characterised in that the ether is methyl tert-butyl ether or dimethoxymethane.

5. Composition according to one of Claims 1 to 4, characterised in that the total quantity of a) is between 0.05 and 5 % by weight of the total mass of the composition, and preferably between 0.2 and 2 %.

6. Composition according to one of Claims 1 to 5, characterised in that the total quantity of b) is between 0.05 and 5 % by weight of the total mass of the composition, and preferably between 2 and 4 %.

EP 0 258 079 B1

7. Composition according to one of Claims 1 to 6, characterised in that the total quantity of c) is between 0.05 and 5 % by weight of the total mass of the composition, and preferably between 1 and 3 %.

8. Composition according to one of Claims 1 to 7, characterised in that a) is propylene oxide, b) is methanol and c) is methyl tert-butyl ether.

9. Process for the removal of photoresist films on printed circuits, characterised in that the said circuits are brought into contact by soaking or spraying with a composition according to one of Claims 1 to 8.

**Patentansprüche**

1. Masse, nützlich zum Entfernen von Photoresistfilmen, dadurch gekennzeichnet, daß sie aus Methylenchlorid und

    (a) mindestens einem Epoxid
    (b) mindestens einem Alkohol und
    (c) mindestens einem Produkt besteht,

    das unter den in Wasser wenig löslichen Ethern und Ketonen ausgewählt ist, wobei die Ether mit 2 bis 12 Kohlenstoffatome und die aliphatischen Ketone 6 bis 10 Kohlenstoffatome enthalten.

2. Masse nach Anspruch 1, dadurch gekennzeichnet, daß das Epoxid unter Propylenoxid und Butylenoxid ausgewählt ist.

3. Masse nach Anspruch 1 oder 2, dadurch gekennzeichnet, daß der Alkohol unter den linearen oder verzweigten Alkoholen mit 1 bis 5 Kohlenstoffatomen ausgewählt und vorzugsweise Methanol ist.

4. Masse nach einem der Ansprüche 1 bis 3, dadurch gekennzeichnet, daß der Ether Methyl-tert.-butylether oder Dimethoxymethan ist.

5. Masse nach einem der Ansprüche 1 bis 4, dadurch gekennzeichnet, daß die Gesamtmenge an a) 0,05 bis 5 Gew.-% der Gesamtmasse und vorzugsweise 0,2 bis 2 % ausmacht.

6. Masse nach einem der Ansprüche 1 bis 5, dadurch gekennzeichnet, daß die Gesamtmenge an b) 0,05 bis 5 Gew.-% der Gesamtmasse und vorzugsweise 2 bis 4 % ausmacht.

7. Masse nach einem der Ansprüche 1 bis 6, dadurch gekennzeichnet, daß die Gesamtmenge an c) 0,05 bis 5 Gew.-% der Gesamtmasse und vorzugsweise 1 bis 3 % ausmacht.

8. Masse nach einem der Ansprüche 1 bis 7, dadurch gekennzeichnet, daß a) Propylenoxid, b) Methanol und c) Methyl-tert.-butylether ist.

9. Verfahren zum Entfernen von Photoresistfilmen auf gedruckten Schaltungen, dadurch gekennzeichnet, daß die Schaltungen durch Eintauchen oder Spritzen mit einer Masse nach einem der Ansprüche 1 bis 8 in Berührung gebracht werden.

7